Europäisches Patentamt

European Patent Office

Office européen des brevets

(19)

(11) **EP 0 999 819 B1**

(12) **EUROPÄISCHE PATENTSCHRIFT**

(45) Veröffentlichungstag und Bekanntmachung des Hinweises auf die Patenterteilung:
**12.11.2003 Patentblatt 2003/46**

(21) Anmeldenummer: **98939505.8**

(22) Anmeldetag: **16.06.1998**

(51) Int Cl.⁷: **A61K 7/00**

(86) Internationale Anmeldenummer:
**PCT/EP98/03615**

(87) Internationale Veröffentlichungsnummer:
**WO 98/057614 (23.12.1998 Gazette 1998/51)**

(54) **PHYSIOLOGISCHE WIRKSTOFFKOMBINATION ZUR FÖRDERUNG DER ABWEHRLEISTUNGEN DER HAUT GEGENÜBER NOXEN**

PHYSIOLOGICAL COMBINATION OF ACTIVE SUBSTANCES TO ENHANCE THE SKIN'S DEFENSES AGAINST NOXIOUS AGENTS

COMBINAISON DE PRINCIPE ACTIF PHYSIOLOGIQUE DESTINEE A STIMULER LES CAPACITES DE DEFENSE DE LA PEAU VIS-A-VIS DE FACTEURS D'AGRESSION

(84) Benannte Vertragsstaaten:
**BE CH DE DK ES FR GB IT LI NL**

(30) Priorität: **17.06.1997 DE 19725405**

(43) Veröffentlichungstag der Anmeldung:
**17.05.2000 Patentblatt 2000/20**

(73) Patentinhaber: **Kramer, Axel, Prof.Dr.med.habil. 17489 Greifswald (DE)**

(72) Erfinder: **Kramer, Axel, Prof.Dr.med.habil. 17489 Greifswald (DE)**

(74) Vertreter:
**Jönsson, Hans-Peter, Dr.Dipl.-Chem. et al Patentanwälte
von Kreisler Selting Werner
Postfach 10 22 41
50462 Köln (DE)**

(56) Entgegenhaltungen:
**WO-A-98/40047          CH-A- 230 366
DE-A- 4 134 888          DE-A- 4 341 001
FR-A- 2 168 202          US-A- 3 384 548**

**Beschreibung**

Die Erfindung betrifft **die Verwendung** einer Wirkstoffkombination zur Herstellung eines Mittels zur Förderung der Abwehrleistung der Haut gegenüber chemischen und physikalischen Irritationen, zum Beispiel bei beruflichen Belastungen, Strapazierung der Haut im Freizeitbereich (Baden, Duschen, Sonneneinwirkung, Haushaltschemikalien, Baustoffe, u.a.) und in der Arbeitsumwelt.

Zum Hautschutz wird seit Jahrzehnten eine Vielzahl von Hautschutzpräparaten eingesetzt, ohne daß ihre Wirksamkeit experimentell belegt ist. Üblicherweise wird der Hautschutz dabei dadurch erreicht, daß der Schutz durch eine Expositionsbarriere ggf. in Verbindung mit einer Erhöhung der Hautfeuchtigkeit, nicht aber durch eine Förderung der Barriere-Funktion und der Abwehrleistung der Haut gegenüber Irritationen auf biochemischem Weg erreicht wird (Elsner P., Wigger-Alberti W. (1997) Der Hautschutz in der Prävention der Berufsdermatosen, Dt. Ärzteblatt 94; 17-1489-A-1492).

Für den Wirkstoff Rhodanid (Thiocyanat) sind als biologische Wirkungen am Hautorgan eine Reihe von Anwendungen experimentell gesichert. Kramer A.: Prüfsystem zur Erfassung der Verträglichkeit antimikrobiell wirksamer Stoffe und Zubereitungen zu episomatischen Applikation durch in-vitro und tierexperimentielle Tests (Episomatiktest) und toxikohygienische Bewertung als Bestandteil krankenhaushygienischer Aufgabenstellungen; Diss. B. Med. Fak. Univ. Greifswald (1985) beschreibt eine beschleunigte Abheilung von Hautwunden. In der DE 41 34 888 A wird die beschleunigte Abheilung von UV induziertem Erythem (Sonnenbrand) beschrieben.

A. Kramer , W. Weuffen, S. Minnich, S. Koch, M. Minnich, H. Below, B. Thürkow und H. Meffert (1990), Förderung der Haarentwicklung durch Thiocyanat beim Meerschweinchen; Dertamto. Mschr. 176: 417-420 beschreiben eine Wachstumsförderung von Hautanhangsgebilden (Haar).

Für den Wirkstoff Harnstoff sind ebenfalls eine Reihe von folgenden Wirkungen bekannt (Wohlrab W. (1989), Bedeutung von Harnstoff in der externen Therapie, Hautarzt 40, Suppl. 9: 35-41), wobei der Anwendungsbereich für die nachfolgend genannten Hamstoffwirkungen jeweils 2 Gew.-% nicht unterschreitet:

Hydratisierung des Stratum corneum, keratolytische Eigenschaften, Beschleunigung der Penetration, Hemmung der epidermalen Proliferation (Basalzellen), geringe antimikrobielle Wirksamkeit, antipruriginöser Effekt, proteo- und mukolytische Aktivität und puffernde Wirkung (Regulation des Hydrolipidmantels).

An therapeutisch nutzbaren Effekten ergeben sich aus diesen Eigenschaften des Harnstoffs (Raab W. (1989) Biochemie, Pharmakologie und Toxikologie von Harnstoff; Hautarzt 40 Suppl.9:23-26):

Hydratisierung der Hornschicht, Keratolyse erkrankter Nägel, Schuppenlösung, Reduzierung überschießender Zellteilungen, Juckreizstillung, Einsparung von Konservierungsmitteln durch antimikrobiellen Effekt und Verbesserung der Penetration eingearbeiteter Pharmaka.

In der modernen Dermatotherapie wird Harnstoff als Monotherapeutikum oder als Zusatzstoff in der Abheilungsphase von Dermatosen erfolgreich angewandt. Als Monotherapeutikum wird Harnstoff bei Ekzemen trockener Haut, bei schuppenden Läsionen, bei Hyperkeratosen und bei vielen anderen chronischen Dermatosen eingesetzt (Swanbeck G. (1989), Harnstoff als Monotherapeutikum bei trockener Haut; Hautarzt 40 Suppl. 9: 42-43)., Harnstoff wird in dermatologischen Externa häufig mit Glukokortikoiden (Drosner M. (1989), Harnstoff in Kombination mit Kortikosteroiden zur Therapie von Ekzemen; Hautarzt 40, Suppl. 9: 47-50), mit Dithranol (Przybilla B., Kaudewitz P., Biber K. (1989), Harnstoff in Kombination mit Dithranol zur Therapie der Psorlasis vulgaris; Hautarzt 40, Suppl.9: 54-57), mit Tretinoin (Müller K.H., Pflugshaupt Ch. (1989); Harnstoff in der Dermatologie; Hautarzt 40, Suppl. 9: 16) und mit Salicylsäure (Gabard B., Bieli E. (1989); Salicylsäure und Harnstoff-mögliche Beeinflussung der keratolytischen Wirkung von Salicylsäure durch Harnstoff; Hautarzt 40, Suppl. 9: 71-73) kombiniert. Bei Neurodermitis, Psoriasis vulgaris und Verhornungsstörungen (Raab loc. cit) wird die regelmäßige Anwendung harnstoffhaltiger Externa zur Rehabilitation der Haut und zur Prophylaxe von Hautläsionen empfohlen, ebenso beim trocken-fettarmen Hautzustand. Harnstoff ist, wie aufgrund seines physiologischen Vorkommens im Organismus (Hibber J.M., Jackson A.A. (1991); Variation in measures of urea kinetics over four years in a single adult; Europ. J. clin. Nutr. 45: 347-351) nicht anders zu erwarten, epidermal gut verträglich. Es sind keine sensibilisierende Potenz oder andere Nebenwirkungen des Harnstoffs nachgewiesen (Ashton H., Frenk E., Stevenson C.J. (1971); Urea as a topical agent; Brit. J. Derm. 84: 194-196; Wozniak K.D. (1975); Hauttestungen verschiedener Hamstofftypen; Derm. Mschr. 161: 687).

In US-A-3,384,548 werden Harnstoff (2 bis 7,5 Gew.-%) und Thiocyanationen (4 Gew.-%) zur Depilation von Haar als Quellmittel eingesetzt.

In der DE 43 41 001 A1 wird ein Harnstoffgehalt von 0,1 bis 10 Gew.-% zur Feuchtigkeitsbindung genannt.

WO 98/40047 betrifft ein Mittel zur Förderung der Mundhygiene und der Mundgesundheit, insbesondere zur Hemmung der Akkumulation bakterieller Plaque auf Zähnen, Zahnersatz und jeweils angrenzenden Oberflächen zur Prophylaxe sowie zur Therapie von Entzündungen und Erkrankungen der Mundschleimhaut, der Gingiva, des Parodontiums sowie zur Kariesprophylaxe. Mittel zur Förderung der Mundhygiene und Mundgesundheit umfassend ionisch gebundene oder freie Thiocyanationen und Carbamid-Perhydrat neben an sich bekannten Hilfs- und Trägerstoffen.

Eine anti-irritative Wirkung im engeren Sinne ist für Harnstoff bisher jedoch nicht bekannt und aus den vorliegenden Befunden nicht abzuleiten.

Der Erfindung liegt die Aufgabe zugrunde, neue wirksame Mittel zur Prophylaxe von Hautschäden durch eine physiologische Hautschutzwirkung auf biochemische Wege zu erreichen, wobei zugleich Heil- und Reparaturvorgänge stimuliert werden, so daß in der Summation ein revitalisierender Hautschutz erreicht wird.

Die Aufgabenstellung wird gelöst durch die Kombination von ionisch gebundenen und/oder freien Thiocyanationen mit Harnstoff neben an sich bekannten Trägerund Hilfsstoffen. Sowohl bei Thiocyanat als auch bei Harnstoff handelt es sich um physiologische Substrate, die im menschlichen Organismus in verschiedenen Kompartimenten vorkommen, die erfindungsgemäß in der Wirkstoffkombination in einem physiologischen Konzentrationsbereich eingesetzt werden.

Gegenstand der Erfindung in einer ersten Ausführungsform ist die Verwendung einer kombination von a) ionisch gebundenen und/oder freien Thiocyanationen und b Harnstoff oder Harnstoffderivaten zur Herstellung eines äußerlich anzuwendenden Mittels zur Förderung der Abwehrleistung der Haut gegenüber chemischen und physikalischen Irritationen, insbesondere bei beruflichen Belastungen, Strapazierungen der Haut im Freizeitbereich und in der Arbeitsumwelt.

Das überraschende Moment der Wirkstoffkombination besteht darin, daß sie im Unterschied zu bekannten Antiphlogistika, die nur erkrankte Gewebe beeinflussen können, bereits bei einer der Irritation vorausgehenden Anwendung diese signifikant in ihrer Ausprägung unterdrücken. Ein derartiges Wirkungsprinzip ist bisher nicht bekannt.

Besonders bevorzugt im Sinne der vorliegenden Erfindung liegen die Thiocyanationen in Form von Alkalimetallsalzen einschließlich der Ammoniumsalze und ihrer Derivate vor. Hierbei handelt es sich somit um dissoziationsfähige Thiocyanate im Gegensatz zu organischen, kovalent gebundenen Thiocyanaten. Besonders bevorzugt im Sinne der vorliegenden Erfindung sind die Alkalimetallsalze der Thiocyanate, ausgewählt aus Natrium-, Kalium- und Ammoniumthiocyanat.

Im Sinne der vorliegenden Erfindung enthalten die Mittel besonders bevorzugt 0,01 Gew.-% bis 1 Gew.-% Thiocyanationen und 0,1 Gew.-% bis 10 Gew.-%, insbesondere bis 1 Gew.-% Harnstoff. Neben den Wirkstoffen Thiocyanat und Harnstoff enthalten die erfindungsgemäßen Mittel übliche Hilfs- und Trägerstoffe für Salben, Cremes, Emulsionen oder Lotionen auf Basis von W/O sowie O/W-Grundlagen oder wässrige Lösungen.

Diese sind beispielsweise aus H.P. Fiedler, Lexikon der Hilfsstoffe für Pharmazie, Kosmetik und angewandte Gebiete 4. Auflage (1996) bekannt, sollen erfindungsgemäß das Verhalten von auf die Haut aufgebrachten Wirkstoffen nicht ändern, wenigstens nicht negativ beeinflussen. Gleiches gilt für die Trägerstoffe. Besonders bevorzugt im Sinne der vorliegenden Erfindung ist das Gewichtsverhältnis von Thiocyanationen zu Harnstoff von 1 : 10 bis 1 : 50.

Wenn im Sinne der vorliegenden Erfindung die Rede von "Harnstoff" ist, so schließt dieser Begriff auch an sich bekannte Harnstoffderivate, wie beispielsweise Monoacetylharnstoff mit ein.

Die erfindungsgemäße Kombination von Thiocyanat mit Harnstoff führte auf der Grundlage additiver Kombinationseffekte zu der überraschenden Hautschutzwirkung der Substanzkombination gegenüber Noxen beziehungsweise den eingangs genannten Belastungen, wobei die bekannte Wirkung von Thiocyanat überraschenderweise durch die Kombination mit Harnstoff verbessert wurde. Mit Hilfe der vorliegenden Erfindung wurde die Möglichkeit eines umfassenden Wirkungsprinzips eines revitalisierenden Hautschutzes verwirklicht.

Eine besondere Ausführungsform der Erfindung besteht in der Verwendung der oben definierten Mittel zur Förderung der Abwehrleistung der Haut gegenüber chemischen und physikalischen Irritationen, insbesondere bei beruflichen Belastungen, Strapazierungen der Haut im Freizeitbereich und in der Arbeitsumwelt. So dient die erfindungsgemäße Wirkstoffkombination insbesondere dem präventiven Schutz vor aggressiven Medien im Berufsalltag ebenso wie beim Umgang mit Schutzmedien gegen äußere Einflüsse auf die Haut.

Ausführungsbeispiele:

**Beispiel 1:**

Der Wirkungsnachweis der Förderung der Abwehrleistung der Haut gegenüber Noxen wurde im Hühnerei-Test an der Chorioallantoismembran (HET-CAM), einem anerkannten Voraussagetest zur Erfassung der Irritationspotenz für Haut und Schleimhaut, geführt.

Für die Prüfung wurden Eier der Rasse "Amerikanisches Weißes Leghorn" (Shaver Starcross 288) eingesetzt. Die Eier wurden 9 d (Bebrütungsbeginn = 1. Tag) in einem Kleinmotorbrüter mit automatischer Wendevorrichtung und automatischer Befeuchtungsregulierung (Typ KMB F/2, EHRET GmbH) bei 37 ± 1 °C und 65 ± 5 % relativer Luftfeuchte inkubiert. Am 9. Tag wurden die Eier zur Festlegung der Belegung durchleuchtet, die nicht belegten Eier aussortiert, die übrigen Eier mit dem stumpfen Pol (Luftblase) nach oben aufrecht gestellt und für weitere 24 h ohne Wenden inkubiert. Am 10 Tag wurde der Eipol mit einer feinen Schere und Pinzette abpräpariert und die Chorioallantoismembran freigelegt.

Geprüft wurden folgende Sachverhalte:

- Reizwirkung der Wirkstoffkombination und ihrer Komponenten
- Schutzwirkung

Hierzu wurden jeweils 0,2 ml der wäßrigen Prüfsubstanzlösung appliziert und nach einem Intervall von 5 min 0,2 ml einer 2 %igen Lösung des Irritanz Natriumdodecylsulfat appliziert. Der Reaktionsausfall wurde mit der Wirkung bei Anwendung von Wasser (Placebo) nach folgendem Score verglichen:

0 keine Reaktion
1 Hyperämie - leicht
2 Hyperämie - mittel
3 Hyperämie - stark
4 Hämorrhagie - vereinzelt
5 Hämorrhagie - häufig
6 Hämorrhagie - massenhaft
7 Koagulation

Parallel dazu wurden als weitere Wirkstoffe Kochsalzlösung in Konzentrationen von 0,05 Gew.-%, 0,5 Gew.-% und 1,0 Gew.-%, Calciumchlorid 0,05 Gew.-%, 0,5 Gew.-% und 5 Gew.-% sowie Sorbitol 0,01 Gew.-%, 0,1 Gew.-% und 1 Gew.-% geprüft.

NaCl, $CaCl_2$ und Sorbitol entfalteten keinen protektiven, insbesondere keinen präventiven Effekt und unterschieden sich in ihrer Wirkung nicht von destilliertem Wasser. Destilliertes Wasser selbst war ebenfalls ohne Einfluß auf die Reizantwort von Natriumdodecylsulfat, wenn dieses 5 min danach appliziert wurde, im Vergleich zur alleinigen Applikation von Natriumdodecylsulfat (Median in beiden Fällen zu allen Einwirkungszeiten identisch).

Sowohl Thiocyanat allein als auch in etwas geringerer Ausprägung Harnstoff allein entfalten eine hautschutzprotektive Wirkung, die von der Kombination beider Wirkstoffe in überraschender Weise signifikant übertroffen wird, wie aus Tabelle 1 hervorgeht.

Tabelle 1:

| Protektive Hautschutzwirkung gemäß dem obigen Score bei nachfolgender Einwirkung der Irritanz 2 % Natriumdodecylsulfat (Intervall zwischen Prüfsubstanzanwendung und Irritanzeinwirkung 5 min) | | | | | |
|---|---|---|---|---|---|
| Beispiele | | Anzahl der untersuchten Eier | Reaktion ($\underline{X}$) nach | | |
| | Prüfsubstanz | | 30 s | 2 min | 5 min |
| Vgl. 1 | Aqua dest. | 96 | 3,8 | 4,9 | 5,6 |
| Vgl. 2 | 0,03 Gew.-% NaSCN | 6 | 2,8 | 3,1 | 3,8 |
| Vgl. 3 | 0,5 Gew.-% Harnstoff | 6 | 2,8 | 3,6 | 4,0 |
| 1 | 0,03 Gew.-% NaSCN + 0,5 Gew.-% Harnstoff | 6 | 1,8 | 2,8 | 3,3 |
| $X = \dfrac{\Sigma \text{ des Reaktionsausfalls pro Ei}}{\text{Eizahl}}$ | | | | | |

**Patentansprüche**

1. Verwendung einer Kombination von a) ionisch gebundenen und/oder freien Thiocyanationen und b) Harnstoff oder Harnstoffderivaten zur Herstellung eines äußerlich anzuwendenden Mittels zur Förderung der Abwehrleistung der Haut gegenüber chemischen und physikalischen Irritationen, insbesondere bei beruflichen Belastungen, Strapazierungen der Haut im Freizeitbereich und in der Arbeitsumwelt.

2. Verwendung nach Anspruch 1, **dadurch gekennzeichnet, daß** man Thiocyanationen in Form von Alkalimetallsalzen, einschließlich Ammonlumsalzen und ihrer Derivate einsetzt.

3. Verwendung nach Anspruch 2, **dadurch gekennzeichnet, daß** man Alkalimetallsalze der Thiocyanate, ausgewählt aus Natrium-, Kalium- und Ammoniumthiocyanat, einsetzt.

4. Verwendung nach Anspruch 1, **dadurch gekennzeichnet, dass** man 0,1 bis 10 g/kg Thiocyanationen, bezogen auf das Mittel, einsetzt.

5. Verwendung nach Anspruch 1, **dadurch gekennzeichnet, dass** man 1,0 bis 100 g/kg Harnstoff, bezogen auf das Mittel einsetzt.

6. Verwendung nach Anspruch 1, **dadurch gekennzeichnet, dass** man 1,0 bis 10 g/kg Harnstoff, bezogen auf das Mittel einsetzt.

7. Verwendung nach Anspruch 1, **dadurch gekennzeichnet, dass** man ein Gewichtsverhältnis von Thiocyanationen zu Harnstoff im Bereich von 1 : 10 bis 1 : 50 einsetzt.

8. Verwendung nach Anspruch 1, **dadurch gekennzeichnet, dass** man Salben, Cremes, Emulsionen, Lotionen und wässrige Lösungen herstellt.

## Claims

1. Use of a combination of a) ionically bound and/or free thiocyanate ions and b) urea or urea derivatives for the preparation of a topical agent for enhancing the skin's defense against chemical and physical irritations, especially from job-related stress, strain on the skin from leisure activities and from the working environment.

2. The use according to claim 1, **characterized in that** said thiocyanate ions are employed in the form of alkali metal salts including ammonium salts and their derivatives.

3. The use according to claim 2, **characterized in that** alkali metal salts of said thiocyanates selected from sodium, potassium and ammonium thiocyanates are employed.

4. The use according to claim 1, **characterized in that** from 0.1 to 10 g of thiocyanate ions per kg of agent is employed.

5. The use according to claim 1, **characterized in that** from 1.0 to 100 g of urea per kg of agent is employed.

6. The use according to claim 1, **characterized in that** from 1.0 to 10 g of urea per kg of agent is employed.

7. The use according to claim 1, **characterized in that** a weight ratio of thiocyanate ions to urea within a range of from 1:10 to 1:50 is employed.

8. The use according to claim 1, **characterized in that** ointments, creams, emulsions, lotions and aqueous solutions are prepared.

## Revendications

1. Utilisation d'une combinaison

   a) d'ions thiocyanate libres et/ou liés par interaction ionique, et
   b) d'urée ou de dérivés d'urée, pour la fabrication d'une composition à application externe, destinée à améliorer la résistance de la peau vis-à-vis d'une irritation chimique ou physique, en particulier dans le cadre professionnel, en cas de stress cutané dans le domaine des loisirs et du travail.

2. Utilisation selon la revendication 1, **caractérisée par le fait que** l'on utilise des ions thiocyanates sous forme de sels de métaux alcalins et de sels d'ammonium et des dérivés de ceux-ci.

3. Utilisation selon la revendication 2, **caractérisée par le fait que** l'on utilise des thiocyanates de métaux alcalins, choisis parmi les sels de sodium et de potassium, et des thiocyanates d'ammonium.

4. Utilisation selon la revendication 1, **caractérisée par le fait que** la composition contient de 0,1 à 10 g d'ions thiocyanates par kg de composition.

**5.** Utilisation selon la revendication 1, **caractérisée par le fait que** la composition contient de 0,1 à 100 g d'urée par kg de composition.

**6.** Utilisation selon la revendication 1, **caractérisée par le fait que** la composition contient de 0,1 à 10 g d'urée par kg de composition.

**7.** Utilisation selon la revendication 1, **caractérisée par le fait que** le rapport en poids des ions thiocyanate à l'urée est compris entre 1 : 10 et 1 : 50.

**8.** Utilisation selon la revendication 1, **caractérisée par le fait que** l'on prépare des pommades, crèmes, émulsions, lotions et des solutions aqueuses.